# EUROPÄISCHE PATENTANMELDUNG

(11) **EP 3 354 316 A1**
(43) Veröffentlichungstag der Anmeldung: **01.08.2018**
(21) Anmeldenummer: 18151591.7
(22) Anmeldetag: 15.01.2018
(51) Int. Cl.: A61N 5/06

(54) **BLAULICHT-THERAPIEGERÄT**

(30) Priorität: 27.01.2017 DE 102017101625
(71) Anmelder: Fairmedic GmbH, 01309 Dresden (DE)
(72) Erfinder: FISCHER, Jörn-Hendrik, 01705 Freital (DE); NIKULSKI, Jendrik, 01705 Freital (DE)
(74) Vertreter: Kaufmann, Sigfrid

(57) **Zusammenfassung**

Die Erfindung betrifft ein Blaulicht-Therapiegerät zur Lichtbehandlung der Haut, aufweisend eine flexible Manschette (1), in welche eine Leuchtdiodenanordnung (2) eingebettet ist. Mittels Kühlkanälen (7), durch welche ein Kühlfluid strömt, ist die Leuchtdiodenanordnung (2) kühlbar. Auslassdüsen (10), die von den Kühlkanälen zu der Behandlungsseite (3) der Manschette (1) verlaufen, erlauben während des Einsatzes des Blaulicht-Therapiegeräts eine zusätzliche aktive Kühlung der behandelten Hautpartie, wobei eine makroskopische Oberflächenstruktur (6) auf der Behandlungsseite (3) das gleichmäßige Entweichen von Kühlfluid aus den Auslassdüsen (10) ermöglicht.

## Beschreibung

Die Erfindung betrifft ein eine flexible Manschette umfassendes Blaulicht-Therapiegerät zur Lichtbehandlung der Haut gemäß Oberbegriff des Anspruchs 1.

Die Bestrahlung der Haut mit Licht hat sich als effektiv zur Behandlung zahlreicher medizinischer Indikationen, wie Herpes, Schuppenflechte oder Akne, erwiesen. Studien zeigten hierbei eine Abhängigkeit der Heilwirkung von der Wellenlänge des eingesetzten Lichtes. Als besonders effektiv zur Behandlung von z. B. Schuppenflechte zeigte sich hierbei blaues Licht.

Zur Licht-Therapie sind fest installierte Lichtbänke, ähnlich einer Sonnenbank, bekannt. Diese Lichtbänke sind jedoch aufgrund ihrer Größe nur stationär an dedizierten Plätzen einsetzbar, wobei aufgrund der großflächigen Bestrahlung eines Patienten stets ein Augenschutz zu tragen ist. Außerdem wird ein Teil der zur Lichterzeugung eingesetzten Energie in unerwünschte Abwärme umgewandelt, was bei Anlagen von dieser Größe eine ausreichend starke Kühleinrichtung notwendig werden lässt.

Zur Umgehung dieser Nachteile wurden kleine, tragbare Geräte entwickelt. Diese können z. B. Handgeräte mit einer Beleuchtungsfläche von weniger als 0.5 m² sein, die mittels eines Bandes am Körper befestigt werden können. Ein derartiges Gerät beschreibt die WO 2009/125338 A1. Nachteilig an diesen Lösungen ist jedoch, dass sich das weitestgehend starre Gerät nicht den Körperformen anpassen kann und nicht zuletzt aufgrund seiner geometrischen Abmessungen unkomfortabel am Körper zu tragen ist.

EP 1 212 118 A1 offenbart ein eine flexible Manschette umfassendes Blaulicht-Therapiegerät, wobei die Lichtquelle in die Manschette integriert ist. Somit kann die Manschette zur Blaulichtbestrahlung betroffener Hautpartien z. B. um den Arm gewickelt werden. Nachteilig an dieser Lösung ist der direkte Hautkontakt, sodass keine Belüftung der Haut möglich ist, die unerwünschte Abwärme bei der Lichterzeugung direkt auf die Haut des Patienten übertragen wird, wobei gegebenenfalls zusätzlich Unverträglichkeiten mit oder Befindlichkeiten gegenüber dem Material der Manschette deren Einsatz behindern.

Aufgabe der Erfindung ist es, diese o. g. Nachteile des Standes der Technik zu beseitigen. Insbesondere soll ein handliches und bequem tragbares Blaulicht-Therapiegerät, umfassend eine auf den betroffenen Körperteil auflegbare, flexible Manschette, geschaffen werden, wobei die unerwünschte Abwärme effektiv abgeführt und die bei einem Patienten aus dem direkten Hautkontakt resultierenden Unannehmlichkeiten abgemildert werden sollen.

Diese Aufgabe wird erfindungsgemäß durch die kennzeichnenden Merkmale des Anspruchs 1 gelöst; weitere vorteilhafte Ausführungen ergeben sich aus den Ansprüchen 2 bis 10.

Ausgegangen wird von einer flexiblen Manschette aus z. B. medizinischem Silikon. In diese Manschette ist eine Leuchtdiodenanordnung zur Abstrahlung von Behandlungsstrahlung im sichtbaren blauen Lichtbereich in Richtung einer Behandlungsseite, d. h. der bei Anwendung des Gerätes mit der Haut des Patienten in Kontakt tretenden Oberfläche, der Manschette integriert. Die Leuchtdiodenanordnung ist innerhalb der Manschette bevorzugt mit einem Abstand zu der Behandlungsseite der Manschette angeordnet, wobei die Manschette zumindest auf dieser Behandlungsseite aus einem für die Behandlungsstrahlung transparentem Material ist. Die Leuchtdiodenanordnung kann aus einer Vielzahl von flächig angeordneten einzelnen Leuchtdioden (LED - lichtemittierende Diode) oder LED-Streifen bestehen. In vorteilhafter Weise beträgt hierbei die Flächendichte der Leuchtdioden auf der Behandlungsseite der Manschette wenigstens 0.25 cm⁻², d. h. wenigstens eine Leuchtdiode auf 4 Quadratzentimeter.

Weiterhin umfasst die Erfindung Mittel zur aktiven Kühlung der Leuchtdiodenanordnung, die in die Manschette unterhalb der Oberseite, d. h. der der Behandlungsseite gegenüberliegenden und bei bestimmungsgemäßer Applikation der Manschette auf die Haut nach außen gerichtete Oberflächenbereich, eingebrachte, von einem Kühlfluid durchströmbare Kühlkanäle, die in einen, bevorzugt an einer Stirnseite der Manschette angeordneten, Fluideingang, d. h. eine Öffnung für den Eintritt des Kühlfluides in die Kühlkanäle der Manschette, münden.

Es kann vorgesehen sein, dass die Manschette, z. B. an ihrer der Stirnseite mit dem Fluideingang gegenüberliegenden Stirnseite, einen Fluidausgang aufweist, in welchen die Kühlkanäle münden, sodass ein durch den Fluideingang in die Kühlkanäle der Manschette strömendes Kühlfluid aus dem Fluidausgang wieder aus der Manschette austreten kann.

Zusätzlich umfasst das Blaulicht-Therapiegerät eine an wenigstens den Fluideingang der Manschette anschließbare oder mit diesem verbundene Fluidenergiemaschine, z. B. einen Kompressor oder eine Mikropumpe.

Erfindungsgemäß sind in die Manschette auf der Behandlungsseite eine Vielzahl kegel- oder kegelstumpfförmiger Ausnehmungen eingebracht, die von der Oberfläche der Behandlungsseite bis in die Kühlkanäle reichen und somit als weitere Fluidausgänge für ein durch die Kühlkanäle strömendes Fluid dienen. Diese Ausnehmungen werden im Folgenden ihrer Funktionsweise entsprechend als Auslassdüsen bezeichnet. Der Innendurchmesser an der Basis dieser kegel- oder kegelstumpfförmigen Auslassdüsen ist klein im Vergleich zum Querschnitt eines Kühlkanals. Außerdem sind die Spitzen der Auslassdüsen zur Behandlungsseite ausgerichtet, d. h., der Querschnitt einer Auslassdüse nimmt in Richtung von dem Kühlkanal zu der Behandlungsseite ab. Somit kann nur ein kleiner Teil eines durch die Kühlkanäle strömenden Kühlfluids durch jede der Auslassdüsen auf der Behandlungsseite aus der Manschette austreten.

Die Oberfläche der Behandlungsseite der Manschette weist ferner eine makroskopische (d. h. mit Dimensionen größer als etwa 1 mm) Oberflächenstruktur aus einer Vielzahl von noppenartigen Erhebungen auf. Diese noppenartigen Erhebungen können aus der Oberfläche herausragende, zueinander beabstandete Kugelsegmente, z. B. Halbkugeln, sein.

Das erfindungsgemäße Blaulicht-Therapiegerät zeigt gegenüber den üblicherweise verwendeten Systemen mehrere Vorteile.

Wesentlicher Vorteil des Blaulicht-Therapiegeräts - neben seiner flexiblen Anpassbarkeit an jedwede anatomische Form - ist, dass dieses eine direkte, aktive Kühlung der unmittelbar bestrahlten Hautpartien ermöglicht. Auf diese Art können auch möglicherweise durch den Hautkontakt verursachte Juckreize oder Befindlichkeiten des Patienten abgemildert werden. Indem die Behandlungsseite der Manschette eine Oberflächenstruktur aufweist, ist ein über deren gesamte Fläche gleichmäßiges Entweichen von Kühlfluid aus den Auslassdüsen ermöglicht.

Durch die Verwendung von Leuchtdioden ist der Energieverbrauch im Vergleich zu traditionellen Lichtquellen reduziert, wobei aufgrund einer wenige Millimeter großen Ausdehnung der Leuchtdioden deren Platzbedarf gering und ihre Anordnung innerhalb der Manschette kaum eingeschränkt ist.

Die Erfindung kann weiter derart ausgebildet sein, dass sich die Kühlkanäle parallel zueinander zwischen einem mit dem Fluideingang verbundenen Kühlfluidverteilkanal und einem mit dem Fluidausgang verbundenen Kühlfluidsammelkanal erstrecken. Hierbei können der Kühlfluidverteilkanal und der Kühlfluidsammelkanal jeweils als ein entlang der Stirnseite verlaufender Kanal ausgebildet sein, zu denen die Kühlkanäle jeweils senkrecht angeordnet sind.

Auch kann vorgesehen sein, dass die kegel- oder kegelstumpfförmigen Auslassdüsen auf der Behandlungsseite jeweils mittels einer als Rückschlagventil wirkenden Verschlusseinrichtung verschließbar sind, sodass ein Fluidstrom nur von den Kühlkanälen in Richtung zu der Behandlungsseite ermöglicht, jedweder Fluidfluss von der Haut in die Kühlkanäle jedoch verhindert ist. Die Rückschlagventile sind z. B. bewegliche, jeweils in die behandlungsseitige Öffnung der Auslassdüsen ragende und diese bei fehlendem Fluidstrom aus den Auslassdüsen dicht verschließende Zungen, die aus demselben Material wie die Behandlungsseite der Manschette, beispielsweise medizinisches Silikon, bestehen können.

Gemäß einer Ausführungsform sind die Leuchtdioden oder LED-Streifen der Leuchtdiodenanordnung innerhalb der Kühlkanäle angeordnet, sodass diese von einem durch die Kühlkanäle strömenden Kühlfluid direkt umspülbar sind.

Gemäß dieser Ausführungsform können die Leuchtdioden in einen in die Kühlkanäle eingebrachten offenporigen Metallschaum eingebettet sein. Der Metallschaum fixiert einerseits die Leuchtdioden innerhalb der Kühlkanäle und transportiert andererseits effizient die Wärme von den Leuchtdioden weg und erlaubt einen verbesserten Wärmetausch mit dem durch den Metallschaum strömenden Kühlfluid. Hierbei kann vorgesehen sein, dass der Metallschaum die Kühlkanäle vollständig ausfüllt, sodass die Leuchtdioden von allen Seiten in Kontakt mit dem Metallschaum stehen. Die Auslassdüsen hingegen sind vorzugsweise frei von Metallschaum.

Weiter kann das erfindungsgemäße Blaulicht-Therapiegerät einen mit dem Fluideingang verbindbaren Medikamentenvorratsbehälter zur Aufnahme eines flüssigen, verflüssigbaren oder zerstäubbaren Wirkstoffes aufweisen. Somit ist es möglich, mit dem durch die Auslassdüsen auf die erkrankten Hautpartien auftreffenden Kühlfluid ein Medikament oder eine reizmildernde Substanz, z. B. als Aerosol, aufzubringen.

Weiter kann die Erfindung derart ausgestaltet sein, dass die Fluidenergiemaschine als MEMS-Bauteil ausgeführt ist. MEMS (Mikro-elektro-mechanische Systeme) ist der Fachbegriff für Mikrosystem-Technik und umfasst mechanisch arbeitende Geräte, deren Abmessungen im Mikrometer- bis Millimeterbereich liegen. Dieses MEMS-Bauteil kann aufgrund seiner geringen Abmessungen direkt in den Fluideingang integriert sein, sodass keine eigenständige Fluidenergiemaschine vor Gebrauch des Blaulicht-Therapiegerätes mit dem Fluideingang zu verbinden ist.

Gemäß einer Ausführungsform umfasst das Blaulicht-Therapiegerät einen Zeitgeber, z. B. eine manuell bedienbare Zeitschaltuhr, mittels dessen die Dauer der Bestrahlung mit Blaulicht vorgebbar ist, sodass nach Ablauf der vorgegebenen Zeitdauer die Leuchtdioden der Leuchtdiodenanordnung automatisch abgeschaltet werden.

Auch kann ein automatischer Überhitzungsschutz vorgesehen sein, der bei Erreichen einer vorgegebenen Grenztemperatur der Manschette, des aus dem Fluidausgang strömenden Kühlfluids oder der Leuchtdiodenanordnung die Stromzufuhr zur Leuchtdiodenanordnung unterbricht. Hierfür kann das Blaulicht-Therapiegerät einen Temperatursensor und eine mit diesem und einer Energieversorgung für die Leuchtdiodenanordnung verbundene Abschalteinrichtung umfassen. Die vorgegebene Grenztemperatur kann fest in der Abschalteinrichtung oder dem Temperatursensor implementiert sein. Abschalteinrichtung und Temperatursensor können auch, beispielsweise in Form eines Bimetallstreifens, als eine bauliche Einheit ausgeführt sein.

Die Erfindung wird nachfolgend anhand von zwei Ausführungsbeispielen näher erläutert; hierzu zeigen in schematischer Darstellung:
- Fig. 1:: eine erste Variante des Blaulicht-Therapiegerätes (Schnitt; Ansicht von der Oberseite);
- Fig. 2:: die erste Variante des Blaulicht-Therapiegerätes in Schrägdraufsicht mit angeschnittener Oberseite;
- Fig. 3:: die erste Variante des Blaulicht-Therapiegerätes im Querschnitt; und
- Fig. 4:: eine zweite Variante des Blaulicht-Therapiegerätes im Querschnitt

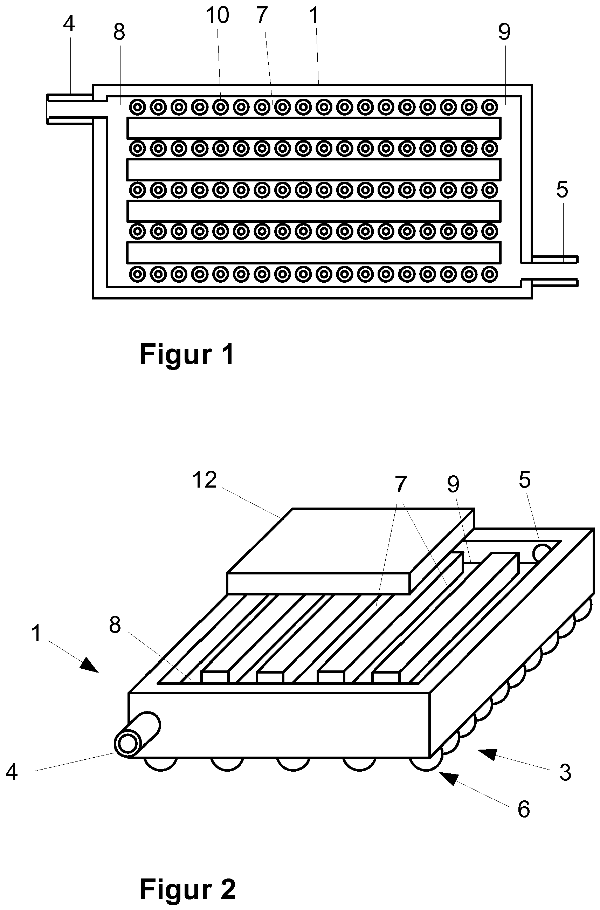

In Fig. 1 ist eine Draufsicht auf die flexible Manschette 1 ohne deren Oberseite dargestellt, sodass die innerhalb der Manschette 1 verlaufenden Kühlkanäle 7 sichtbar sind. Die Kühlkanäle 7 verlaufen parallel zueinander und erstrecken sich von dem Kühlfluidverteilkanal 8 zu dem Kühlfluidsammelkanal 9. Der Kühlfluidverteilkanal 8 ist mit dem Fluideingang 4, der Kühlfluidsammelkanal 9 mit dem Fluidausgang 5 verbunden.

In die flexible Manschette 1 sind die Auslassdüsen 10 eingebracht, die sich von den Kühlkanälen 7 bis zur Behandlungsseite (in Fig. 1 nicht dargestellt) erstrecken.

In der Schrägdraufsicht gemäß Figur 2 ist die nach unten gerichtete Behandlungsseite 3 der Manschette 1 zu sehen, welche die noppenartige Oberflächenstruktur 6 aufweist.

Die Oberseite 12 der Manschette ist nur angeschnitten dargestellt, um den Blick auf die Kühlkanäle 7 freizugeben.

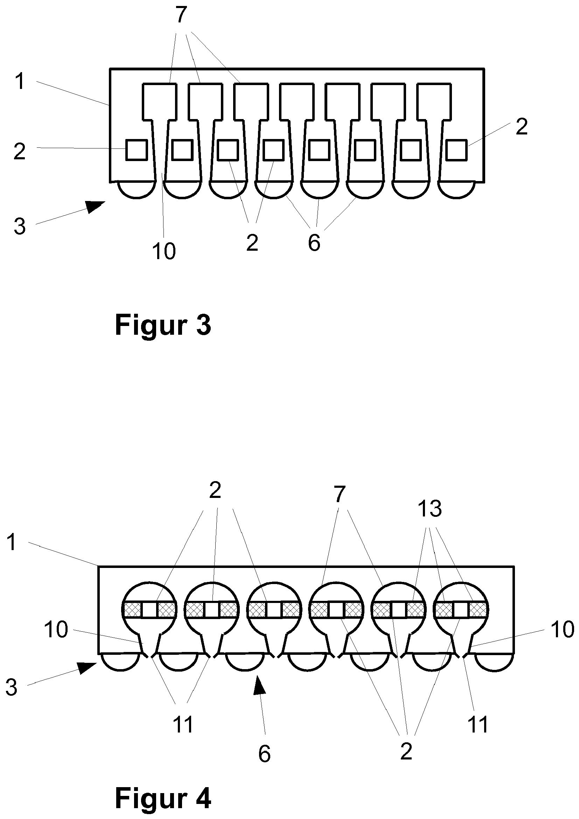

Figur 3 zeigt einen Querschnitt durch die Manschette 1. Die Leuchtdiodenanordnung 2 ist in Form von einzelnen Leuchtdioden (LED) ausgebildet, die innerhalb der Manschette 1 zwischen der Behandlungsseite 3 und den Kühlkanälen 7 angeordnet sind. Die zwischen den LEDs angeordneten kegelstumpfförmigen Auslassdüsen 10 erlauben einen Kühlfluidfluss aus den Kühlkanälen 7 auf die Behandlungsseite 3 der Manschette 1.

In Figur 4 ist eine weitere Ausführung der Manschette 1 des Blaulicht-Therapiegerätes im Querschnitt dargestellt. Bei dieser Ausführungsform sind die Leuchtdioden der Leuchtdiodenanordnung 2 innerhalb der Kühlkanäle 7 angeordnet. Zwei jeweils seitlich zu den Leuchtdioden innerhalb der Kühlkanäle verlaufende Quader aus offenporigem Metallschaum 13 fixieren die Leuchtdioden innerhalb der Kühlkanäle 7. Indem die Kühlkanäle 7 nur auf einem Teil ihres Querschnittes mit dem Metallschaum 13 gefüllt sind, ist der Strömungsquerschnitt für ein durch die Kühlkanäle 7 strömendes Fluid nur wenig eingeschränkt. Auf der Behandlungsseite 3 der Manschette 1 weisen die Auslassdüsen 10 jeweils das Rückschlagventil 11 auf, welches hier jeweils mittels vier flexibler Klappen realisiert ist.

### Liste der verwendeten Bezugszeichen

- 1: flexible Manschette
- 2: Leuchtdiodenanordnung
- 3: Behandlungsseite
- 4: Fluideingang
- 5: Fluidausgang
- 6: Oberflächenstruktur
- 7: Kühlkanal
- 8: Kühlfluidverteilkanal
- 9: Kühlfluidsammelkanal
- 10: kegelstumpfförmige Ausnehmung / Auslassdüse
- 11: Rückschlagventil
- 12: Oberseite
- 13: offenporiger Metallschaum

## Patentansprüche

1. Blaulicht-Therapiegerät zur Lichtbehandlung der Haut, aufweisend
- eine flexible Manschette (1) mit einer Behandlungsseite (3) und einer der Behandlungsseite (3) gegenüberliegenden Oberseite (12), wobei die Oberfläche der Behandlungsseite (3) eine makroskopische Oberflächenstruktur (6) aufweist;
- eine innerhalb der Manschette (1) befindliche Leuchtdiodenanordnung (2) zur Abstrahlung von Behandlungsstrahlung in Richtung der Behandlungsseite (3) der Manschette (1), sowie
- Mittel zur aktiven Kühlung der Leuchtdiodenanordnung (2), umfassend in die Manschette (1) unterhalb der Oberseite (12) eingebrachte Kühlkanäle (7), die in einen an einer Stirnseite der Manschette (1) angeordneten Fluideingang (4) münden,
**dadurch gekennzeichnet, dass**
- die Manschette (1) einen an ihrer der Stirnseite mit dem Fluideingang (4) gegenüberliegenden Stirnseite einen mit den Kühlkanälen (7) verbundenen Fluidausgang (5) aufweist,
- das Blaulicht-Therapiegerät eine an wenigstens den Fluideingang (4) der Manschette (1) anschließbare oder mit diesem verbundene Fluidenergiemaschine umfasst,
- die Oberflächenstruktur (6) eine Vielzahl von noppenartigen Erhebungen umfasst, und
- in die Manschette eine Vielzahl kegel- oder kegelstumpfförmiger Auslassdüsen (10) eingebracht sind, die sich von der Behandlungsseite (3) bis in die Kühlkanäle (7) erstrecken, wobei die Spitzen der kegel- oder kegelstumpfförmigen Auslassdüsen (10) zur Behandlungsseite (3) weisen.

2. Blaulicht-Therapiegerät nach Anspruch 1, **dadurch gekennzeichnet, dass** sich die Kühlkanäle (7) parallel zueinander zwischen einem mit dem Fluideingang (4) verbundenen Kühlfluidverteilkanal (8) und einem mit dem Fluidausgang (5) verbundenen Kühlfluidsammelkanal (9) erstrecken.

3. Blaulicht-Therapiegerät nach Anspruch 2, **dadurch gekennzeichnet, dass** der Kühlfluidverteilkanal (8) und der Kühlfluidsammelkanal (9) jeweils als ein entlang der Stirnseite der Manschette (3) verlaufender Kanal ausgebildet sind, zu denen die Kühlkanäle (7) jeweils senkrecht angeordnet sind.

4. Blaulicht-Therapiegerät nach einem der vorstehenden Ansprüche, **dadurch gekennzeichnet, dass** die kegel- oder kegelstumpfförmigen Auslassdüsen (10) auf der Behandlungsseite (3) jeweils mittels eines Rückschlagventils (11) verschlossen sind, das einen Fluidstrom nur von den Kühlkanälen (7) in Richtung zu der Behandlungsseite (3) ermöglicht.

5. Blaulicht-Therapiegerät nach Anspruch 4, **dadurch gekennzeichnet, dass** die Rückschlagventile (11) jeweils in die behandlungsseitige Öffnung der Auslassdüsen (10) ragende Zungen sind, die aus demselben Material wie die Behandlungsseite (3) der Manschette (1) bestehen.

6. Blaulicht-Therapiegerät nach einem der vorstehenden Ansprüche, **dadurch gekennzeichnet, dass** die Leuchtdiodenanordnung (2) eine Vielzahl von Leuchtdioden umfasst, wobei die Leuchtdioden innerhalb der Kühlkanäle (7) angeordnet sind.

7. Blaulicht-Therapiegerät nach Anspruch 6, **dadurch gekennzeichnet, dass** die Leuchtdioden in einen in die Kühlkanäle (7) eingebrachten offenporigen Metallschaum (13) eingebettet sind.

8. Blaulicht-Therapiegerät nach einem der vorstehenden Ansprüche, **dadurch gekennzeichnet, dass** es einen mit dem Fluideingang (4) verbindbaren Medikamentenvorratsbehälter zur Aufnahme eines flüssigen, verflüssigbaren oder zerstäubbaren Wirkstoffes aufweist.

9. Blaulicht-Therapiegerät nach einem der vorstehenden Ansprüche, **dadurch gekennzeichnet, dass** die Leuchtdiodenanordnung (2) eine Vielzahl von Leuchtdioden umfasst, wobei die Flächendichte der Leuchtdioden auf der Behandlungsseite (3) der Manschette (1) wenigstens 0.25 cm⁻² beträgt.

10. Blaulicht-Therapiegerät nach einem der vorstehenden Ansprüche, **dadurch gekennzeichnet, dass** die Fluidenergiemaschine ein als Mikrosystem-Technik-Bauelement ausgeführter Mini-Kompressor oder Mikropumpe ist.
